Europäisches Patentamt

European Patent Office

Office européen des brevets

(19)

(11) Veröffentlichungsnummer : **0 389 840 B1**

## (12) EUROPÄISCHE PATENTSCHRIFT

(45) Veröffentlichungstag der Patentschrift :
08.09.93 Patentblatt 93/36

(51) Int. Cl.⁵ : **A61M 1/16**

(21) Anmeldenummer : **90104422.2**

(22) Anmeldetag : **08.03.90**

(54) **Hämodialysegerät mit automatischer Einstellung des Dialysierflüssigkeitsflusses.**

(30) Priorität : **25.03.89 DE 3909967**

(43) Veröffentlichungstag der Anmeldung :
**03.10.90 Patentblatt 90/40**

(45) Bekanntmachung des Hinweises auf die
Patenterteilung :
**08.09.93 Patentblatt 93/36**

(84) Benannte Vertragsstaaten :
**DE ES FR GB GR IT SE**

(56) Entgegenhaltungen :
**EP-A- 0 089 003**
**EP-A- 0 122 604**
**GB-A- 2 196 756**
**US-A- 4 739 492**

(56) Entgegenhaltungen :
**CHEMIE-ING.-TECHN., Band 47, April 1975,
Seiten 334-342, Weinheim, DE; A. PASTER-
NACK.** "**Verfahrenstechnische Aspekte bei
der Entwicklung und dem Betrieb künstlicher
Nieren**"

(73) Patentinhaber : **Fresenius AG**
**Gluckensteinweg 5**
**D-61350 Bad Homburg (DE)**

(72) Erfinder : **Polaschegg, Hans-Dietrich, Dr.**
**Grünwiesenweg 9**
**D-6370 Oberursel 4 (DE)**

(74) Vertreter : **Dr. Fuchs, Dr. Luderschmidt Dr.
Mehler, Dipl.-Ing Weiss Patentanwälte
Postfach 46 60, Abraham-Lincoln-Strasse 7
D-65036 Wiesbaden (DE)**

EP 0 389 840 B1

**Beschreibung**

Die Erfindung bezieht sich auf ein Hämodialysegerät mit einem Hämodialysator, der blutseitig eine Blutpumpe, die Blut von einem Patienten zum Dialysator fördert und dialysierflüssigkeitsseitig eine Dialysierflüssigkeitspumpe, die Dialysierflüssigkeit zum Dialysator fördert, aufweist.

Derartige Hämodialysegeräte sind in mannigfaltigen Ausführungsformen bekanntgeworden, z.B. aus der DE-OS 36 36 995.

Traditionell werden diese Hämodialysegeräte mit einem konstanten und vom Benutzer nicht veränderbaren Dialysierflüssigkeitsfluß von 500 ml/min betrieben. Neuere Geräte erlauben die gestufte, manuelle Einstellung auch anderer Dialysierflüssigkeitsflüsse, z.B. 300, 500 und 800 ml/min. Höhere Dialysierflüssigkeitsflüsse sind insbesondere zur Erzielung einer hohen Austauschleistung (clearance) bei hohen Blutflüssen von 400 ml/min oder darüber erforderlich.

Die Kosten einer Dialysebehandlung mit derartigen Hämodialysegeräten setzen sich aus den
- Fixkosten der Behandlungsstation und des Dialysegerätes (Abschreibung sowie Reparatur)
- Kosten des Verbrauchsmaterials: Dialysator, Blutschlauchsystem, Kanüle, Tupfer, Desinfektionsmittel, Dialysekonzentrat, aufbereitetes Wasser und Energie sowie
- Personalkosten

zusammen. Die Bemühungen, die Behandlungskosten so gering wie möglich zu halten, finden - bei gegebenen Dialysegeräten - an den vorgenannten Fix- und den Personalkosten keinen großen Spielraum. Ansatzpunkte für eine Ersparnis bieten die Kosten des Verbrauchsmaterials.

Es ist bekannt (siehe J.E. Sigdell, B.Tersteegen, Artificial Organs, 10 (3) Seite 219-225, 1986), daß bei den weit verbreiteten Kapillar-Dialysatoren bei einem Verhältnis Blutfluß: Dialysierflüssigkeitsfluß von 1:2 eine gegenüber den oben genannten "Standardbedingungen" von 500 ml/min Dialysierflüssigkeitsfluß nur vernachlässigbar geringe Verringerung der Austauschleistung auftritt. Obwohl dieser Effekt bereits seit längerem bekannt ist und auch aus der Produktbeschreibung von Dialysatorherstellern hervorgeht, andererseits der Kostendruck insbesondere in den USA gut dokumentiert ist und zur Wiederverwendung von Produkten geführt hat, die an sich als Einmalartikel konzipiert waren, sind bisher keine Maßnahme zur Einsparung von Dialysekonzentrat, Wasser und Energie getroffen worden, da eine manuelle Veränderung des Dialysierflüssigkeitsflusses in vielen Fällen nicht möglich und nach Auffassung der Fachwelt auch zu aufwendig und mit der Gefahr der Fehlbedienung verbunden ist.

Der Erfindung liegt die Aufgabe zugrunde, das eingangs bezeichnete Hämodialysegerät so auszubilden, daß der Verbrauch an Dialysekonzentrat, aufbereitetem Wasser sowie Energie minimiert wird, um die Kosten einer Dialysebehandlung ohne Beeinträchtigung der Behandlungsqualität zu mindern.

Die Lösung dieser Aufgabe gelingt gemäß der Erfindung dadurch, daß eine Steuereinrichtung vorgesehen ist, der eingangsseitig ein von der Größe des Blutflusses abgeleitetes Signal zugeführt ist und die ausgangsseitig ein Signal zur Einstellung des Dialysierflüssigkeitsflusses als Funktion des Blutflusses abgibt.

Durch die erfindungsgemäße selbsttätige Steuerung wird erreicht, daß bei der Hämodialyse der Dialysierflüssigkeitsfluß stets als Funktion des Blutflusses selbsttätig eingestellt wird. Auf diese Weise ist mit Vorteil sichergestellt, daß zu jedem Zeitpunkt der Hämodialysebehandlung einschließlich der Vorbereitungs- und Abschlußphase, ein für die Behandlung ausreichender, gleichwohl nicht zu hoher Dialysierflüssigkeitsfluß selbsttätig eingestellt wird. Das mit der erfindungsgemäßen selbsttätigen Steuerung ausgestattete Hämodialysegerät zeichnet sich dadurch mit Vorteil durch niedrige Verbrauchskosten aus.

Es ist an sich bekannt, daß bei Hämodialysegeräten der eingangs genannten Art die üblichen Sensoren und Stellglieder mit einem Rechner verbunden sind, der den Ablauf selbsttätig steuert bzw. regelt. Die Steuerung des Dialysierflüssigkeitsflusses abhängig von dem Blutfluß ist im bekannten Fall nicht vorgesehen, noch ist ein entsprechender Hinweis zu entnehmen.

An Hand eines in der Zeichnung dargestellten Ausführungsbeispieles wird die Erfindung bzw. die sie ausgestaltenden Merkmale näher beschrieben.

Es zeigen:

Figur 1    ein Blockschaltbild eines üblichen Hämodialysegerätes mit der erfindungsgemäßen Steuerung,
Figur 2    eine Ausführungsform der in der erfindungsgemäßen Steuerung verwendeten Steuerfunktion mit linearen Abschnitten.

Die Figur 2 zeigt eine konventionelle Hämodialysevorrichtung mit einem Hämodialysator 10, der blutseitig einen Einlaß 11 und einen Auslaß 12 sowie dialysierflüssigkeitsseitig einen Einlaß 13 und einen Auslaß 14 aufweist. In dem Dialysierflüssigkeitskreis ist eine Dialysierflüssigkeitspumpe 20 vorgesehen, die Dialysierflüssigkeit aus einer nicht näher dargestellten Dialysierflüssigkeitsquelle fördert.

Der Dialysierflüssigkeitskreis weist in bekannter Weise einen Leitfähigkeitssensor 21 und einen Temperatursensor 22 auf. Es ist ferner ein Dialysierventil 23 vorgesehen, mit dem die Dialysierflüssigkeit auf den Dialysator 10 aufgeschaltet werden kann. Mit einem Bypassventil 24 kann der Dialysierflüssigkeitsfluß vom Dialysator weggenommen werden. Der Dialysierflüssigkeitskreis weist ferner einen Drucksensor 25 zur Messung des Dialysierflüssigkeitsdruckes stromab des Dialysators auf.

Im blutseitigen Kreislauf befindet sich eine Blutpumpe 30, die Blut von einem nicht dargestellten Patienten zum Dialysator fördert. Stromauf der Blutpumpe 30 ist ein arterieller Drucksensor 31 angeordnet. Im Blutkreislauf befindet sich weiterhin stromab des Dialysators 10 ein venöser Blasenfänger 32, an dem ein Luftdetektor 33 die Detektion von Luft und Blutschaum erlaubt. Stromab des Blasenfängers ist eine venöse Absperrklemme 34 zum Abschließen des extrakorporalen Kreislaufes vom Patienten im Falle des Auftretens eines gefährlichen Zustandes angeordnet. Der Blutkreislauf wird vervollständigt durch einen optischen Detektor 35, der die Unterscheidung von Kochsalzlösung und Blut im Schlauch stromab des venösen Blasenfängers 32 erlaubt, sowie durch einen venösen Rücklaufdrucksensor 36, der mit dem Blasenfänger 32 verbunden ist.

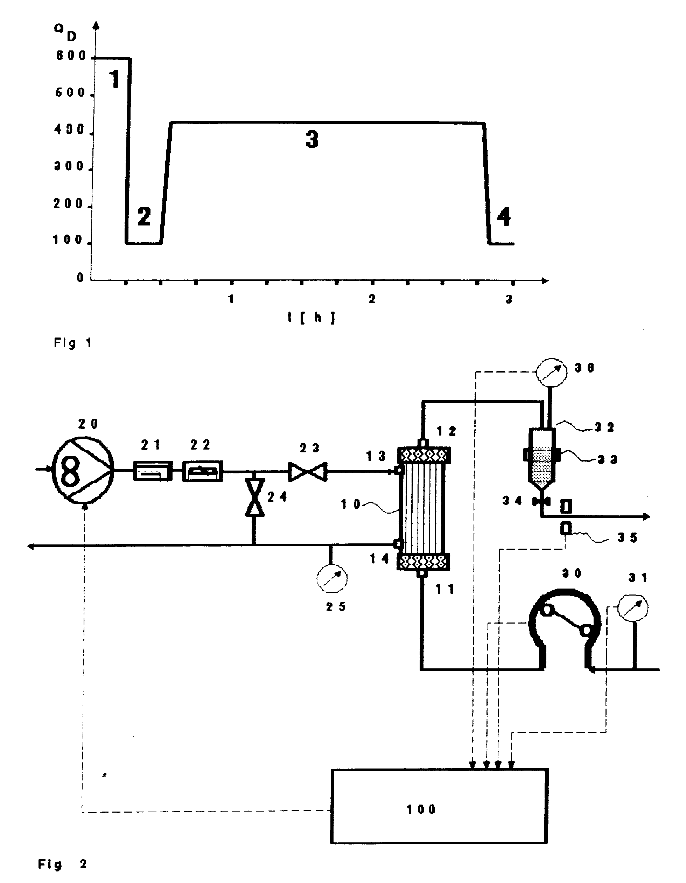

Der erfindungsgemäße selbsttätige Steuerkreis ist in der Steuereinrichtung 100 enthalten. Dieser Steuereinrichtung wird eingangsseitig ein Signal zugeführt, das von der Größe der Blutflusses abgeleitet ist. Dieses Signal wird zweckmäßig von der eingestellten Förderrate der Blutpumpe 30 abgeleitet, die sich beispielsweise aus der Drehzahl der Blutpumpe 30 herleitet. Das Ausgangssignal des Steuerkreises, welches eine vorgegebene Funktion des Blutflusses ist, dient zur selbsttätigen Einstellung des Dialysierflüssigkeitsflusses. Zu diesem Zwecke ist der Ausgang des Steuerkreises 100 auf die Dialysierflüssigkeitspumpe 20 geschaltet, und zwar auf den Eingang des unterlagerten Steuer/Regelkreises zur Einstellung der Förderrate dieser Pumpe. Die Steuereinrichtung 100 enthält weiterhin Einrichtungen, die es erlauben zu unterscheiden, in welchem der Zustände 1 bis 4 gemäß Figur 1 sich das Hämodialysergerät befindet, wobei diese Stufen später an Hand der Figur 1 noch näher erläutert werden. Zu diesem Zweck werden auf den Eingang der Steuereinrichtung 100 auch die Ausgangssignale des arteriellen Drucksensors 31, des venösen Drucksensors 36 und des optischen Detektors 35 geschaltet, wie dies durch die strichlierten Signalleitungen dargestellt ist.

Die Steuereinrichtung 100 kann neben dem erfindungsgemäßen Steuerkreis auch eine übliche Steuereinheit zur Steuerung und Überwachung der Hämodialyse enthalten. In diesem Fall sind alle anderen in Figur 2 gezeichneten Elemente gleichfalls durch Signalleitungen mit der Steuereinheit 100 verbunden zu denken.

Der Aufbau und die Einstellung der Steuereinrichtung 100 ist so getroffen, daß das den Dialysierflüssigkeitsfluß einstellende Ausgangssignal der selbsttätigen Steuerung eine vorgegebene Funktion des vom Blutfluß abgeleiteten Eingangssignales ist, die nach vorgegebenen Kriterien aus dem Clearance-Leistungsfeld (Clearance in Abhängigkeit von dem Dialysierflüssigkeits- und Blutfluß) abgeleitet wird. Diese vorgegebene Funktion ist im einfachsten Fall eine lineare Funktion. Sie kann auch die Form eines Polynoms bzw. durch ein numerisches Datenfeld vorgegeben werden, welches in einer Matrix in dem Steuerkreis abgelegt ist, die zu jedem Blutfluß eines bestimmten Dialysators jenen Dialysierflüssigkeitsfluß angibt, bei dem ein bestimmter Prozentsatz der Clearance bei unendlichem Dialysierflüssigkeitsfluß (z.B. 95 %) erreicht wird. In diesem Fall ist die Steuerfunktion in einer sogenannten Tafel abgelegt.

Die in der Steuereinrichtung 100 eingebrachte Steuerfunktion, die den Dialysierflüssigkeitsfluß $Q_D$ steuert, kann sich aus mehreren zeitlichen Abschnitten zusammensetzen. Diese Abschnitte sind in Figur 1 symbolisch wiedergegeben. Im ersten Abschnitt erfolgt ein Freidialysieren eines mit Desinfektionsmittel gefüllten (wiederverwendeten) Dialysators. Auf der Blutseite rezirkuliert Kochsalzlösung mit einer hohen Rate. Die Dialysierflüssigkeitsseite wird mit maximaler Rate (z.B. 800 ml/min) betrieben.

Im zweiten Abschnitt ist das Hämodialysegerät an den Patienten angeschlossen. Der Dialysierflüssigkeitsfluß wird auf einen konstanten, kleinen Wert gesetzt, um eine zu starke Abkühlung des extrakorporalen Kreislaufs zu vermeiden.

Im dritten Abschnitt erfolgt die eigentliche Dialyse. Der Dialysierflüssigkeitsfluß $Q_D$ wird in Abhängigkeit vom Blutfluß $Q_B$ entsprechend der allgemeinen Funktion $Q_D=f(Q_B)$ gesteuert.

Im vierten Abschnitt wird die Dialyse beendet. Der Dialysierflüssigkeitsfluß wird auf einen konstanten Wert eingestellt. Bei diesem Wert beharrt die Einstellung dann im "stand-by"-Betrieb bis zur nächsten Behandlung oder zum nächsten Verfahrensschritt.

Die automatische Erkennung des Zustandes (Abschnittes) der Dialyse erfolgt aufgrund der folgenden Signale.

Zustand 1: Zum Freispülen des Dialysators stellt der Anwender die Blutpumpe 30 manuell auf eine hohe Geschwindigkeit (typisch 400 ml/min). Der optische Detektor 35 zeigt das Signal "hell", d.h. das Vorhandensein von Kochsalz im Schlauchsystem an. Der Druck am arteriellen Drucksensor 31 ist > -50 mmHg, wogegen der Druck am venösen Rücklaufdrucksensor 36 < 50 mmHg ist. Letztere Druckwerte sind typisch, können sich jedoch in Abhängigkeit vom verwendeten Schlauchsystem bzw. Spülverfahren ändern. Sie sind jedenfalls nach einmaliger "in-vitro" Erprobung im engen Intervall festlegbar. Da während des Freispülens es gelegentlich auch zur Schaumbildung im Blasenfänger 32 kommt, kann diese Signalkombination auch zur sicheren Überbrückung des Luft-

3

detektors 35 herangezogen werden.

Zustand 2: Beim Anschließen des Gerätes an den Patienten wird die Förderrate der Blutpumpe 30 am Ende von Zustand 1 auf 0 gestellt und langsam erhöht. Der optische Detektor 35 zeigt weiterhin das Signal "hell".

Zustand 3: Der optische Detektor 35 zeigt während des Dialysiervorganges das Signal "dunkel", das die Anwesenheit von Blut signalisiert.

Zustand 4: Die Blutpumpe 30 wird am Ende des Zustandes 3 auf 0 gestellt und die Förderrate der Blutpumpe 30 auf einen kleinen Wert eingestellt. Der optische Detektor 35 zeigt dabei das Signal "dunkel".

Da die Wärmeenergiebilanz des Patienten von der über den Dialysator zugeführten bzw. über ihn abgeführten Wärmeenergie abhängt und diese wiederum von der Menge und der Temperatur der Dialysierflüssigkeit bestimmt wird, wird zweckmäßig die erfindungsgemäße Steuerung durch eine flußabhängige Dialysierflüssigkeit-Temperaturregelung erweitert.

Da überlicherweise die Dialysierflüssigkeittemperatur innerhalb des Dialysegerätes gemessen und geregelt wird, es aber nach dieser Meßstelle zur Energieabgabe zur Umgebung kommt, weicht die eingestellte Dialysierflüssigkeitstemperatur von jener am Dialysatoreingang 13 ab. Eine vom Dialysierflüssigkeitsfluß abhängige Temperaturregelung wird daher die allgemeine Form haben:

$$T_{Regel} = g \ (T_{soll}, Q_D, Q_B).$$

Da der Dialysierflüssigkeitsfluß $Q_D$ als Funktion des Blutflusses $Q_B$ geregelt wird, kann diese allgemeine Regel umgeformt werden in:

$$T_{Regel} = g \ (T_{soll}, Q_B).$$

Mit dieser Beziehung kann der Energietransfer unabhängig von der Dialysierflüssigkeitsveränderung konstant gehalten werden. Die Regelung nach dieser Beziehung ist überflüssig, wenn durch Temperatursensoren stromab und stromauf des Dialysators 10 im extrakorporalen Kreislauf der Energietransfer direkt gemessen wird.

Eine einfache Regelung des Energietransfers im Dialysator 10 ist auch möglich, wenn die Temperatur stromauf und stromab des Dialysators 10 im Dialysierflüssigkeitskreislauf gemessen wird.

Die Funktion $Q_D=f \ (Q_B)$ wird im einfachsten Fall die Form:

$Q_D=a$ für $Q_B >= 0$ und $<= 100$ ml/min

und

$Q_D = b*Q_B$ für 100 ml/min $< Q_B$ annehmen, wobei

a so eingestellt wird, daß es nicht zu einer zu starken Abkühlung im extrakorporalen Kreislauf kommt (typisch etwa 50 -100 ml/min) und

b der Charakteristik des Dialysators entsprechend gewählt wird.

## Patentansprüche

1. Hämodialysegerät mit einem Hämodialysator (10), der blutseitig eine Blutpumpe (30), die Blut von einem Patienten zum Dialysator fördert und dialysierflüssigkeitsseitig eine Dialysierflüssigkeitspumpe (20), die Dialysierflüssigkeit zum Dialysator fördert, aufweist, dadurch gekennzeichnet, daß eine Steuereinrichtung (100) vorgesehen ist, der eingangsseitig ein von der Größe des Blutflusses abgeleitetes Signal zugeführt ist, und die ausgangsseitig ein Signal zur Einstellung des Dialysierflüssigkeitsflusses als Funktion des Blutflusses abgibt.

2. Hämodialysegerät nach Anspruch 1, dadurch gekennzeichnet, daß das von der Größe des Blutflusses abgeleitete Signal von der Pumpgeschwindigkeit (Förderrate) der Blutpumpe (30) abgeleitet ist, und das Ausgangssignal der Steuereinrichtung (100) auf die Dialysierflüssigkeitspumpe (20) geschaltet ist.

3. Hämodialysegerät nach Anspruch 1 oder 2, dadurch gekennzeichnet, daß der Aufbau und die Einstellung der Steuereinrichtung (100) so getroffen ist, daß das den Dialysierflüssigkeitsfluß einstellende Ausgangssignal der Steuereinrichtung eine vorgegebene Funktion des vom Blutfluß abgeleiteten Eingangssignales ist, die nach vorgegebenen Kriterien aus dem Clearance-Leistungsfeld (Clearance in Abhängigkeit vom Dialysierflüssigkeitsfluß und Blutfluß) abgeleitet ist.

4. Hämodialysegerät nach Anspruch 3, dadurch gekennzeichnet, daß die vorgegebene Funktion eine lineare Funktion ist.

5. Hämodialysegerät nach Anspruch 3, dadurch gekennzeichnet, daß die vorgegebene Funktion ein Polynom ist.

6. Hämodialysegerät nach Anspruch 3, dadurch gekennzeichnet, daß die vorgegebene Funktion durch ein numerischen Datenfeld gebildet wird, welches in einer Matrix in der Steuereinrichtung (100) abgelegt ist, die zu jedem Blutfluß eines bestimmten Dialysators jenen Dialysierflüssigkeitsfluß angibt, bei dem ein bestimmter Prozentsatz der Clearance bei unendlichem Dialysierflüssigkeitsfluß (z.B. 95 %) erreicht wird.

7. Hämodialysegerät nach einem der Ansprüche 3 bis 6, dadurch gekennzeichnet, daß der Steuerkreis durch einen Mikroprozessor gebildet wird.

8. Hämodialysegerät nach einem der Ansprüche 1 bis 7, dadurch gekennzeichnet, daß ergänzend zur Steuereinrichtung (100) eine blutflußabhängige Dialysierflüssigkeits- Temperaturregelung vorgesehen ist.

9. Hämodialysegerät nach Anspruch 8, dadurch gekennzeichnet, daß blutseitig im extrakorporalen Kreislauf stromab und stromauf des Dialysators (10) Temperatursensoren zur Istwerterfassung für die Temperaturregelung vorgesehen sind.

10. Hämodialysegerät nach Anspruch 8, dadurch gekennzeichnet, daß dialysierflüssigkeitsseitig stromab und stromauf des Dialysators (10) Temperatursensoren zur Istwerterfassung für die Temperaturregelung vorgesehen sind.

## Claims

1. A hemodialysis apparatus with a hemodialyser (10) comprising on the blood side a blood pump (30) to convey blood from a patient to the dialyser and on the dialysis solution side a dialysis solution pump (20) to convey dialysis solution to the dialyser, characterized in that a control device (100) is provided whereby a signal derived from the magnitude of the blood flow is fed to said device on the input side and whereby said device gives a signal on the output side as a function of the blood flow in order to adjust the dialysis solution flow.

2. The hemodialysis apparatus according to claim 1, characterized in that the signal taken from the magnitude of the blood flow is derived from the pump speed (pumping rate) of the blood pump (30) and the output signal of the control device (100) is connected to the dialysis solution pump (20).

3. The hemodialysis apparatus according to claim 1 or 2, characterized in that the control device (100) is so constructed and regulated that the output signal of the control device which regulates the dialysis solution flow is a predetermined function of the input signal derived from the blood flow, said function being derived according to the predetermined criteria from the area of the clearance performance field (clearance as a function of dialysis solution flow and blood flow).

4. The hemodialysis apparatus according to claim 3, characterized in that the predetermined function is a linear function.

5. The hemodialysis apparatus according to claim 3, characterized in that the predetermined function is a polynomial.

6. The hemodialysis apparatus according to claim 3, characterized in that the predetermined function is formed by a numerical data field which is filed in a matrix in said control device (100) which, for every blood flow rate of a defined dialyser, gives the dialysis solution flow rate at which a certain percentage of the clearance (e.g. 95%) is reached, when the dialysis solution flow rate is infinite.

7. The hemodialysis apparatus according to any of claims 3 to 6, characterized in that the control circuit is formed by means of a microprocessor.

8. The hemodialysis apparatus according to any of claims 1 to 7, characterized in that a dialysis solution and temperature regulation means dependent on the blood flow rate is provided as a supplemental device to the control device (100).

9. The hemodialysis apparatus according to claim 8, characterized in that on the blood side in the extracorporeal circuit temperature detectors are provided upstream and downstream from the dialyser (10) in order to measure the actual values for the temperature controlling.

10. The hemodialysis apparatus according to claim 8, characterized in that on the dialysis side temperature detectors are provided upstream and downstream from the dialyser (10) in order to measure the actual values for the temperature controlling.

## Revendications

1. Appareil d'hémodialyse comportant un hémodialyseur (10), qui comporte, côté sang, une pompe à sang (30), qui entraîne le sang d'un patient jusqu'au dialyseur, et, côté liquide de dialyse, une pompe à liquide de dialyse (20), qui entraîne un liquide de dialyse jusqu'au dialyseur, caractérisé par le fait qu'il est prévu un dispositif de commande (100), auquel est envoyé, côté entrée, un signal dérivé de la valeur du débit sanguin, et qui délivre, côté sortie, un signal pour le réglage du débit du liquide de dialyse, en fonction du débit sanguin.

2. Appareil d'hémodialyse selon la revendication 1, caractérisé en ce que le signal, qui est dérivé de la valeur du débit sanguin, est dérivé de la vitesse (débit de refoulement) de la pompe à sang (30), et le signal de sortie du dispositif de commande (100) est envoyé à la pompe à liquide de dialyse (20).

3. Appareil d'hémodialyse selon la revendication 1 ou 2, caractérisé en ce que l'agencement et le réglage du dispositif de commande (100) sont exécutés de telle sorte que le signal de sortie, qui règle le débit du liquide de dialyse, du dispositif de commande est une fonction prédéterminée du signal d'entrée, qui est dérivé du débit sanguin et qui est dérivé, selon des critères prédéterminés, de la gamme de puissance de clearance (clearance en fonction du débit du liquide de dialyse et du débit sanguin).

4. Appareil d'hémodialyse selon la revendication 3, caractérisé en ce que la fonction prédéterminée est une fonction linéaire.

5. Appareil d'hémodialyse selon la revendication 3, caractérisé en ce que la fonction prédéterminée est un polynôme.

6. Appareil d'hémodialyse selon la revendication 3, caractérisé en ce que la fonction prédéterminée est formée par un ensemble numérique de données, qui est mémorisé dans une matrice dans le dispositif de commande (100), qui indique, pour chaque débit sanguin d'un dialyseur déterminé, le débit du liquide de dialyse, pour lequel un pourcentage déterminé de clearance est atteint pour un débit infini du liquide de dialyse (par exemple 95 %).

7. Appareil d'hémodialyse selon l'une des revendications 3 à 6, caractérisé en ce que le circuit de commande est formé par un microprocesseur.

8. Appareil d'hémodialyse selon l'une des revendications 1 à 7, caractérisé en ce qu'à titre de complément du dispositif de commande (100), il est prévu une régulation liquide de dialyse - température, qui dépend du débit sanguin.

9. Appareil d'hémodialyse selon la revendication 8, caractérisé en ce que côté sang, il est prévu des capteurs de température servant à détecter la valeur réelle pour la régulation de température, dans le circuit extra-corporel, en aval et en amont du dialyseur (10).

10. Appareil d'hémodialyse selon la revendication 8, caractérisé en ce que côté liquide de dialyse, des capteurs de température sont prévus en aval et en amont du dialyseur (10) pour réaliser une détection de valeurs réelles pour la régulation de la température.

Fig 1

Fig 2